(19) Europäisches Patentamt
European Patent Office
Office européen des brevets

(11) Publication number: **0 194 881 B1**

(12) # EUROPEAN PATENT SPECIFICATION

(45) Date of publication of patent specification: **30.01.91**

(21) Application number: **86301806.5**

(22) Date of filing: **13.03.86**

(51) Int. Cl.$^5$: **C 08 F 220/18,** C 09 J 123/00, A 61 L 15/58 // (C08F220/18, 220:12, 222:24)

(54) **Emulsion polymers.**

(30) Priority: **13.03.85 GB 8506436**

(43) Date of publication of application: **17.09.86 Bulletin 86/38**

(45) Publication of the grant of the patent: **30.01.91 Bulletin 91/05**

(84) Designated Contracting States: **AT BE CH DE FR GB IT LI NL SE**

(56) References cited:
**GB-A-1 220 476**
**GB-A-1 430 136**

**CHEMICAL ABSTRACTS, vol. 94, no. 5, March 1981, Columbus, OH (US); p. 94, no. 67377n**

(73) Proprietor: **Smith & Nephew plc**
**2, Temple Place Victoria Embankment**
**London WC2R 3BP (GB)**

(72) Inventor: **Howes, John Gordon Bernard**
**Larond Heath Road**
**Hertford Heath Hertfordshire (GB)**

(74) Representative: **Cole, William Gwyn, Dr.**
**Smith and Nephew Research Limited Gilston Park**
**Harlow Essex CM20 2RQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 194 881 B1**

Courier Press, Leamington Spa, England.

**Description**

The present invention relates to emulsion polymer adhesives and more particularly to emulsion polyacrylate adhesives which are suitable for application to the skin, and to methods of manufacturing the same.

It is known in the art to form polymers by emulsion polymerisation. Such processes are preferred because they avoid the use of solvents which may be environmentally undesirable. The emulsion polymerisation process employs a surfactant to form a stable emulsion of monomers and other ingredients and to prevent coagulation of the polymer emulsion after it has formed. Conventionally a non-polymerisable surfactant is used. However, the residual surfactant in the polymer can be extracted by water and so renders the polymer sensitive to water. To avoid this problem the use of polymerisable surfactants has been proposed. For example, British Patent No. 1,430,136 discloses emulsion polymerised polymers as industrial adhesives suitable for use on masking tapes and lists copolymerisable surfactants suitable for this purpose. British Patent No. 1,220,476 discloses inter alia copolymerisable surfactants having the general formula:

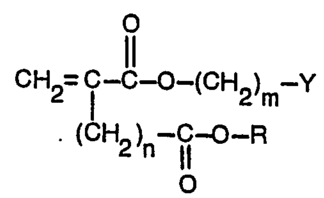

in which m is an integer from 2 to 4, n is an integer 1 or 2, R is a $C_1$ -$C_{22}$ alkyl radical and Y is -$S(O_2)OM$ where M is hydrogen, ammonia or an alkali metal. Such surfactants may be employed in emulsion polymerisation reactions with other vinylic compounds to prepare emulsion polymers which are useful as coatings on substrates such as textiles or plastics to provide antistatic properties or as dye assistants.

I have now found that by employing copolymerisable surfactants selected from the above group and having the formula:

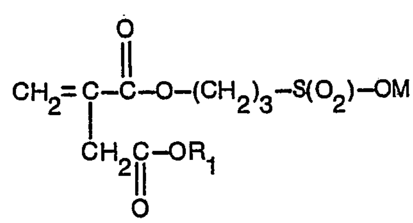

in which $R_1$ is a $C_{10}$ to $C_{14}$ alkyl radical and M is hydrogen, ammonia or an alkali metal, in emulsion polymerisation reactions with one or more copolymerisable acrylic monomers then emulsion polymers are obtained which have adhesive properties and are suitable for application to the skin.

Accordingly the present invention provides an emulsion adhesive polymer comprising residues of acrylic monomers containing 80 to 98.5% by weight of residues of alkyl esters of acrylic acid in which the alkyl group contains from 3 to 12 carbon atoms, from 0.1 to 20% by weight of residues of monoesters of methacrylic acid and 0.1 to 5% by weight of residues of a compound of the formula (I)

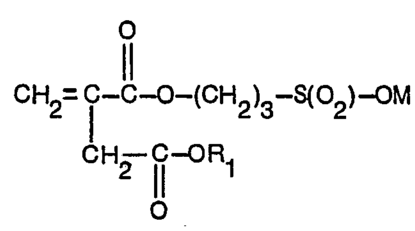

(I)

in which $R_1$ is a $C_{10}$ - $C_{14}$ alkyl radical and M is hydrogen, ammonia or an alkali metal.

Compounds of formula (I) will form stable emulsions with acrylic monomers and will prevent coagulation of any such emulsions during an emulsion polymerisation process. The residues of compound (I) form part of the polymer formed by the process. The polymer produced by the emulsion polymerisation reaction will be an adhesive polymer, that is an acrylic adhesive polymer.

Adhesive polymers of this type may be pressure sensitive adhesive polymers. Such pressure sensitive adhesives may be used on skin, for example in surgical or medical dressings. In order to maintain good

adhesion to skin it is especially desirable that such adhesives are not water sensitive (in that water does not significantly affect their adhesive properties). Neither of the aforementioned British Patents disclose pressure sensitive adhesives for use on skin, for example in surgical or medical dressings.

Aptly compounds of formula (I) will be in the form of an ammonia or alkali metal salt and more suitably will be in the form of the sodium or potassium salt. In a preferred form the compound of formula (I) will be in the form of the sodium salt.

Suitably the alkyl radical will contain from 10 to 14 carbon atoms and more suitably will contain from 11 to 13 carbon atoms and preferably will be an alkyl radical which contains 12 carbon atoms.

In the preparation of preferred adhesive polymers, therefore, in the compound of formula (I) $R_1$ is an alkyl radical containing 12 carbon atoms and M is sodium, that is the preferred compound is sodium mono-lauryl itaconoxypropane sulphonate.

The emulsion adhesive polymer of the invention may suitably contain 0.1 to 5% by weight and preferably 0.1 to 1% by weight of residues of a compound of formula (I) or a salt thereof.

In apt polymers of the invention the acrylic polymer component consists mainly of acrylic residues for which the monomer is an alkyl ester of acrylic acid, in which the residue contains 2 to 20 carbon atoms. Favoured pressure sensitive adhesive forming alkyl acrylate monomers are alkyl esters of acrylic acid in which the alkyl group contains 3 to 12 carbon atoms and preferably 4 to 9 carbon atoms. Alkyl acrylates of this type include n-butyl acrylate, 2-ethylhexyl acrylate and other octyl acrylates. Favourable monomers include n-butyl acrylate and 2-ethylhexyl acrylate.

Aptly these acrylic residues will be derived from a mixture of two monomer species in roughly equal proportions. The adhesive polymers of the invention may advantageously contain residues of other acrylic monomers as hereinafter described. In these instances the acrylic residues may form 80 to 98.5% by weight of the polymer and preferably will form from 88 to 94% by weight of the polymer.

The pressure sensitive adhesive polymers of the present invention may advantageously contain residues of other acrylic monomers. Suitable monomers include acrylic esters comprising monoesters such as optionally hydroxylated or alkoxylated or alkyl esters of methacrylic acid. Preferred other acrylic monomers are monoesters of methacrylic acid selected from the group consisting of lower alkyl, hydroxylated or alkoxylated alkyl esters, for example methyl methacrylate, n-butyl methacrylate, hydroxyethyl methacrylate and methoxyethyl methacrylate. Preferred monomers include hydroxyethyl methacrylate, methyl methacylate and n-butyl methacrylate. Also suitable but not preferred are esters such as glycidyl methacrylate.

Such residues may form 0.1 to 20% by weight of the polymer and more suitably up to 12% by weight and preferably from 5 to 12% by weight of the polymer. The most apt proportion of such residues will depend to some extent on the particular monomer species, the monomer species of the major components, the emulsion polymerisation conditions and the desired physical properties of the pressure sensitive adhesives. Thus hydroxyethyl methacrylate residues may be present from 0.3 to 5% by weight of the polymer and lower alkyl methacrylate residues may be present as 1 to 15% by weight of the polymer.

A suitable pressure sensitive adhesive polymer comprises 36 to 50% by weight of n-butyl acrylate residues, 39 to 59% by weight of 2-ethylhexyl acrylate residues, 1 to 15% by weight of lower alkyl methacrylate residues, 0.3 to 5% by weight of hydroxyethyl methacrylate residues and 0.1 to 5% by weight of residues of a compound of formula (I), e.g. a salt of mono-lauryl itaconoxypropane sulphonic acid. Lower alkyl when used herein means an alkyl radical containing from 1 to 6 carbon atoms.

A favoured pressure sensitive adhesive polymer comprises 39 to 45% by weight of n-butyl acrylate residues, 47 to 49.5% by weight of 2-ethylhexyl acrylate residues, 5 to 10% by weight of lower alkyl methacrylate residues, 0.8 to 3% by weight of hydroxyethyl methacrylate residues and 0.1 to 1% by weight of residues of a compound of formula (I), e.g. a salt of mono-lauryl itaconoxypropane sulphonic acid.

A preferred pressure sensitive adhesive polymer comprises 44.5% by weight of n-butyl acrylate residues, 49.5% by weight of 2-ethylhexyl acrylate residues, 5% by weight of n-butyl acrylate residues, 1% by weight of hydroxyethyl methacrylate residues and 0.2% by weight of sodium mono-lauryl itaconoxypropane sulphate.

The pressure sensitive adhesives of the invention can be used for adhesive surgical and medical dressings and therefore adhesive surgical and medical dressings employing an adhesive of this invention are an important aspect of this invention.

The adhesive of the invention may therefore be coated onto any of the well known backing materials which find use in the medical or surgical fields. Thus the backing may be, for example, a conventional nonwoven fabric, woven fabric, knit, paper or synthetic film backing. Favoured backings include porous polyvinyl chloride film, polyurethane film, integral nets and the like. Suitable backing materials for moisture vapour permeable adhesive dressings are disclosed in British Patent No 1280631 and in European Patent No 50935 and backings which permit passage of tissue or wound exudate therethrough include nonwoven fabrics, woven fabrics, knits, nets and apertured films.

The backing material may be of any desired shape to provide adhesive coated sheet materials as adhesive tapes, strips, wound dressings, surgical drapes or the like.

The emulsion pressure-sensitive adhesive polymer may be applied to the backing by conventional methods, for example by coating an aqueous dispersion of the emulsion which has been suitably thickened

onto a silicone-coated release paper, drying the adhesive and transfer coating onto the appropriate backing material.

The compound of formula (I) may be formed by the processes described in British Patent No 1220476 which is incorporated herein by cross-reference. The preferred copolymerisable surfactant, sodium mono-lauryl itaconoxypropane sulphonate may be prepared in a similar manner to that described in Example 1 of British Patent No 1220476.

The adhesive polymers of the invention can be prepared by a process which comprises polymerising as an emulsion containing 80 to 98.5% by weight of alkyl esters of acrylic acid in which the alkyl group contains from 3 to 12 carbon atoms, from 0.1 to 20% by weight of monoesters of methacrylic acid and 0.1 to 5% of residues of a compound of formula (I) as herein before described in the presence of a free radical catalyst and a metal ion chelating agent, preferably polyphosphate.

The emulsion may conveniently be made by initially forming an aqueous solution containing the surfactant and a heavy metal chelating agent. If present one of the minor monomers may be added to this solution, for example hydroxyethyl methacrylate may be in this solution. the remaining monomers may be premixed and added to the aqueous solution gradually over a period of from 15 minutes to 1 hour while the aqueous mixture is being stirred at high-shear. The resulting emulsion may be left to deaerate. The resulting monomer emulsion typically has a solids content in the range of 15 to 70% by weight and preferably 50 to 60% by weight.

The resulting monomer emulsion may then be added to an aqueous solution of free radical catalyst, for example ammonium persulphate, and polymerisation effected by heating, typically at 80° to 95°C, under an inert atmosphere such as nitrogen or carbon dioxide. Advantageously, the monomer emulsion may be added to the catalyst solution over a period of time during the polymerisation reaction. The resultant polymer emulsion may be cooled and stored until required.

The polyacrylate adhesive emulsions of the invention prior to use may be thickened using a conventional polyacrylate thickener, for example Primal ASE 60 (Trade Mark) neutralised with ammonium hydroxide. The adhesive can be coated onto a suitable substrate by a direct or transfer coating process using conventional coating techniques.

## Example 1

Preparation of Emulsion Polyacrylate Adhesive

Water (65 g) was placed in an open-topped beaker. The head of a Silverson mixer with a high shear head was introduced into a the water and the water mixed during the addition of sodium tripolyphosphate (0.02 g), sodium mono-lauryl itaconoxypropane sulphonate (0.17 g) and hydroxy ethyl methacrylate (0.85 g). A separate mixture of 2-ethylhexyl acrylate (42 g), n-butyl acrylate (38 g) and n-butyl methacrylate (4.25 g) was prepared. This second mixture was added over 20 minutes to the stirred contents of the beaker. When the addition was complete the mixture was stored for a further 15 minutes and then the mixer switched off. The mixture was allowed to deaerate for 30 minutes.

Water (60 g) was placed in a reaction flask fitted with a stirrer and having means of heating the contents of the flask. The water was heated to 82°C in the stirring and then ammonium persulphate (0.000442 g) was added. The monomer emulsion prepared above was added to the flask over 3 hours whilst stirring and maintaining the temperature at 85 to 90°C.

The resultant polymer emulsion was cooled to 40°C before being transferred to a storage jar.

## Example 2

Preparation of Emulsion Polyacrylate Adhesive

An emulsion polymer was prepared in a similar manner to that described in Example 1 except that 4.25 g of methyl methacrylate were used in place of the n-butyl methacrylate.

## Example 3

Preparation of Emulsion Polyacrylate Adhesives

An emulsion polymer was prepared in a similar manner to that described in Example 1 except that 8.5 g of n-butyl methacrylate and 33.75 g of n-butyl acrylate were used in place of the quantities given in Example 1.

## Example 4

Preparation of Surgical Adhesive Tape

The polyacrylate adhesive emulsion prepared as described in Example 1 was thickened by the addition of a thickening agent Primal ASE 60 (trade mark, available from Rohm and Haas) neutralised with ammonium hydroxide and an antioxidant Nonox WSL (trade mark) was also added (0.6% by weight based on the weight of emulsion). This mixture was coated onto a silicone coated release paper (Steralese, trade mark, available from Sterling Coated Papers Limited) by means of a blade over flat bed coater and dried in an air circulating oven at a temperature of 110° ± 5°C to give a dried pressure sensitive adhesive coating with a weight per unit area of 40 $g/m^2$. The adhesive coating was transferred to either a polyester film or to an integral net backing (see Example 2 of British Patent No 1531715) and cut into 2.5 cm wide surgical tapes. The resulting surgical tape adhered well to human skin under dry and wet conditions and onto sweaty skin.

**Claims**

1. An emulsion adhesive polymer comprising residues of acrylic monomers containing 80 to 98.5% by weight of residues of alkyl esters of acrylic acid in which the alkyl group contains from 3 to 12 carbon atoms, from 0.1 to 20% by weight of residues of monoesters of methacrylic acid and 0.1 to 5% by weight of residues of a compound of the formula (I),

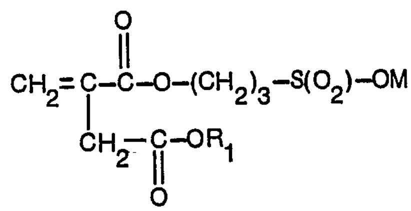

(I)

in which $R_1$ is a $C_{10}$ - $C_{14}$ alkyl radical and M is hydrogen, ammonia or an alkali metal.

2. An emulsion adhesive polymer according to claim 1 in which the compound of formula (I) is a sodium or potassium salt and $R_1$ is an alkyl radical which contains from 11 to 13 carbon atoms.

3. An emulsion adhesive polymer according to claim 2 in which the compound of the formula (I) is sodium mono-lauryl itaconoxypropane sulphonate.

4. An emulsion adhesive polymer according to claim 1 in which the polymer contains from 88 to 94% by weight of residues of a mixture of n-butyl acrylate and 2-ethylhexyl acrylate monomer.

5. An emulsion adhesive polymer according to claim 1 in which the polymer contains from 0.3 to 5% by weight of residues of hydroxyethyl methacrylate monomer.

6. An emulsion adhesive polymer according to claim 1 in which the polymer contains from 1 to 15% by weight of residues of methacrylate monomer of an alkyl radical containing from 1 to 6 carbon atoms.

7. An emulsion adhesive polymer according to claim 1 which is a pressure sensitive adhesive polymer which comprises 36 to 50% by weight of n-butyl acrylate residues, 39 to 59% by weight of 2-ethyl hexyl acrylate residues, 1 to 15% by weight of methacrylate of alkyl radical containing from 1 to 6 carbon atoms residues, 0.3 to 5% by weight of hydroxyethyl methacrylate residues and 0.1 to 5% by weight of residues of a salt of mono-lauryl itaconoxypropane sulphonic acid.

8. An emulsion adhesive polymer according to claim 7 in which the pressure sensitive adhesive polymer comprises 39 to 45% by weight of n-butyl acrylate residues, 47 to 49.5% by weight of 2-ethylhexyl acrylate residues, 5 to 10% by weight of methacrylate of alkyl radical containing from 1 to 6 carbon atoms residues, 0.8 to 3% by weight of hydroxyethyl methacrylate residues and 0.1 to 1% by weight of sodium mono-lauryl itaconoxypropane sulphonate.

9. A process for the preparation of an emulsion adhesive polymer which process comprises polymerising as an emulsion acrylic monomers containing 80 to 98.5% by weight of alkyl esters of acrylic acid in which the alkyl group contains from 3 to 12 carbon atoms, from 0.1 to 20% by weight of monoesters of methacrylic acid selected from the group consisting of alkyl radicals containing from 1 to 6 carbon atoms, hydroxylated or alkoxylated alkyl esters and 0.1 to 5% of residues of a compound of the formula (I),

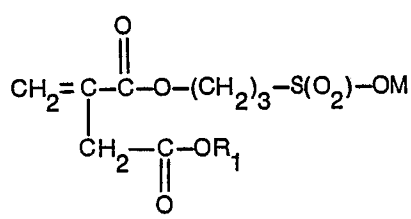

(I)

in which $R_1$ is a $C_{10}$ - $C_{14}$ alkyl radical and M is hydrogen, ammonia or an alkali metal, in the presence of a free radical catalyst and a metal ion chelating agent.

10. A process according to claim 9 in which the metal ion chelating agent is sodium polyphosphate.

11. An adhesive surgical or medical dressing comprising an emulsion adhesive polymer according to claim 1.

**Patentansprüche**

1. Haftendes Emulsionspolymer, umfassend Acrylmonomeren-Reste, welche 80 bis 98,5 Gew.-% Acrylsäurealkylester-Reste, in welchen die Alkylgruppe 3 bis 12 Kohlenstoffatome enthält, 0,1 bis 20 Gew.-% Methacrylsäuremonoester-Reste und 0,1 bis 5 Gew.-% Reste einer Verbindung der Formel (I) enthalten,

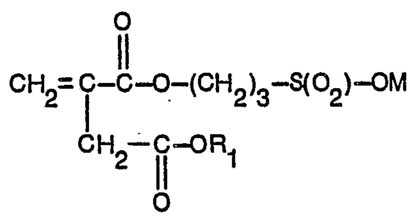
(I)

in welcher $R_1$ ein $C_{10}$-$C_{14}$ Alkylradikal ist und M Wasserstoff, Ammoniak oder ein Alkalimetall ist.

2. Haftendes Emulsionspolymer gemäß Anspruch 1, in welchem die Verbindung der Formel (I) ein Natrium- oder Kaliumsalz ist und $R_1$ ein Alkylradikal ist, welches 11 bis 13 Kohlenstoffatome enthält.

3. Haftendes Emulsionspolymer gemäß Anspruch 2, in welchem die Verbindung der Formel (I) Natrium-monolaurylitaconoxypropansulfonat ist.

4. Haftendes Emulsionspolymer gemäß Anspruch 1, in welchem das Polymer 88 bis 94 Gew.-% eines Gemisches aus n-Butylacrylat- und 2-Ethylhexylacrylatmonomer-Resten enthält.

5. Haftendes Emulsionspolymer gemäß Anspruch 1, in welchem das Polymer 0,3 bis 5 Gew.-% Hydroxyethylmethacrylatmonomer-Reste enthält.

6. Haftendes Emulsionspolymer gemäß Anspruch 1, in welchem das Polymer 1 bis 15 Gew.-% Methacrylatmonomer-Reste eines 1 bis 6 Kohlenstoffatome enthaltenden Alkylradikals enthält.

7. Haftendes Emulsionspolymer gemäß Anspruch 1, welches ein druckempfindliches, haftendes Polymer ist, das 36 bis 50 Gew.-% n-Butylacrylatreste, 39 bis 59 Gew.-% 2-Ethylhexylacrylatreste, 1 bis 15 Gew.-% Methacrylat-Reste eines 1 bis 6 Kohlenstoffatome enthaltenden Alkylradikals, 0,3 bis 5 Gew.-% Hydroxyethylmethacrylat-Reste und 0,1 bis 5 Gew-% Reste eines Monolaurylitaconoxypropansulfonsäure-Salzes enthält.

8. Haftendes Emulsionspolymer gemäß Anspruch 7, in welchem das druckempfindliche haftende Polymer 39 bis 45 Gew.-% n-Butylacrylatreste, 47 bis 49,5 Gew.-% 2-Ethylhexylacrylatreste, 5 bis 10 Gew.-% Methacrylatreste eines 1 bis 6 Kohlenstoffatome enthaltenden Alkylradikals, 0,8 bis 3 Gew.-% Hydroxyethylmethacrylatreste und 0,1 bis 1 Gew.-% Natrium-monolaurylitaconoxypropansulfonat enthält.

9. Verfahren zur Herstellung eines haftenden Emulsionspolymers, welches das Polymerisieren von Acrylmonomeren, welche 80 bis 98,5 Gew.-% Acrylsäurealkylester, in welchen die Alkylgruppe 3 bis 12 Kohlenstoffatome enthält, 0,1 bis 20 Gew.-% Methacrylsäuremonoester, ausgewählt aus der aus 1 bis 6 Kohlenstoffatome enthaltende Alkylradikale aufweisenden, hydroxylierten oder alkoxylierten Alkylestern bestehenden Gruppe, und 0,1 bis 5 Gew.% Reste einer Verbindung der Formel (I) enthalten,

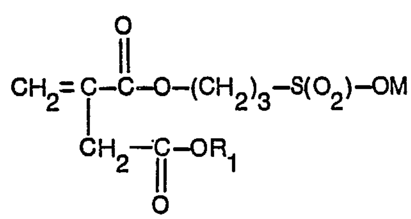
(I)

in welcher $R_1$ ein $C_{10}$-$C_{14}$ Alkylradikal ist und M Wasserstoff, Ammoniak oder ein Alkalimetall ist, in Anwesenheit eines Freiradikalkatalysators und eines Metallionen chelatierenden Mittels als Emulsion umfaßt.

10. Verfahren gemäß Anspruch 9, in welchem das Metallionen chelatierende Mittel Natriumpolyphosphat ist.

11. Chirurgischer oder medizinischer Haftverband umfassend ein haftendes Emulsionspolymer gemäß Anspruch 1.

**Revendications**

1. Polymère adhésif en émulsion, caractérisé en ce qu'il comprend des résidus de monomères acryliques contenant 80 à 98,5% en poids de résidus d'esters alkyliques d'acide acrylique dans lesquels le groupe alkyle contient de 3 à 12 atomes de carbone, de 0,1 à 20% en poids de résidus de monoesters d'acide méthacrylique et 0,1 à 5% en poids de résidus d'un composé de formule (I):

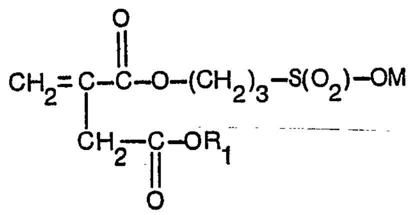

(I)

dans laquelle $R_1$ est un groupe alkyle en $C_{10-14}$ et M est un atome d'hydrogène, de métal alcalin ou un groupe ammonium.

2. Polymère adhésif en émulsion suivant la revendication 1, caractérisé en ce que le composé de formule (I) est un sel de sodium ou de potassium et $R_1$ est un radical alkyle qui contient de 11 à 13 atomes de carbone.

3. Polymère adhésif en émulsion suivant la revendication 2, caractérisé en ce que le composé de formule (I) est le mono-lauryl itaconoxypropane sulfonate de sodium.

4. Polymère adhésif en émulsion suivant la revendication 1, caractérisé en ce que le polymère contient de 88 à 94% en poids de résidus d'un mélange d'acrylate de n-butyle et d'acrylate de 2-éthylhexyle monomères.

5. Polymère adhésif en émulsion suivant la revendication 1, caractérisé en ce que le polymère contient de 0,3 à 5% en poids de résidus de méthacrylate d'hydroxyéthyle monomère.

6. Polymère adhésif en émulsion suivant la revendication 1, caractérisé en ce que le polymère contient de 1 à 15% en poids de résidus de méthacrylate monomère d'un radical alkyle contenant 1 à 6 atomes de carbone.

7. Polymère adhésif en émulsion suivant la revendications 1, caractérisé en ce qu'il est un polymère adhésif sensible à la pression compenant 36 à 50% en poids de résidus d'acrylate de n-butyle, 39 à 59% en poids de résidus d'acrylate de 2-éthylhexyle, 1 à 15% en poids de résidus de méthacrylate du radical alkyle contenant 1 à 6 atomes de carbone, 0,3 à 5% en poids de résidus de méthacrylate d'hydroxyéthyle et 0,1 à 5% en poids de résidus d'un sel d'acide monolauryl itaconoxypropane sulfonique.

8. Polymère adhésif en émulsion suivant la revendication 7, caractérisé en ce que le polymère adhésif sensible à la pression comprend 39 à 45% en poids de résidus d'acrylate de n-butyle, 47 à 49,5% en poids de résidus d'acrylate de 2-éthylhexyle, 5 à 10% en poids de résidus de méthacrylate du radical alkyle contenant de 1 à 6 atomes de carbone, 0,8 à 3% en poids de résidus de méthacrylate d'hydroxyéthyle et 0,1 à 1% en poids de mono lauryl itaconoxypropane sulfonate de sodium.

9. Procédé pour la préparation d'un polymère adhésif en émulsion, caractérisé en ce qu'il comprend la polymérisation en émulsion de monomères acryliques contenant 80 à 98,5% en poids d'esters alkyliques d'acide acrylique dans lesquels le groupe alkyle contient de 3 à 12 atomes de carbone, de 0,1 à 20% en poids de monoesters d'acide méthacrylique choisis parmi les esters alkyliques hydroxylés ou alcoxylés dans lesquels les radicaux alkyles contiennent de 1 à 6 atomes de carbone et 0,1 à 5% de résidus d'un composé de formule (I):

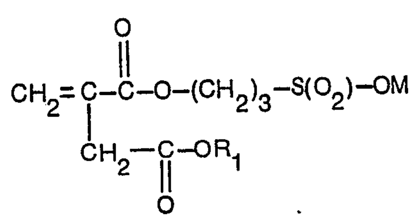

(I)

dans laquelle $R_1$ est un radical alkyle en $C_{10-14}$ et M est un atome d'hydrogène, de métal alcalin ou un groupe ammonium, en présence d'un catalyseur de radicaux libres et d'un agent chélatant les ions métalliques.

10. Procédé suivant la revendication 9, caractérisé en ce que l'agent chélatant les ions métalliques est un polyphosphate de sodium.

11. Pansement adhésif chirurgical ou médical, caractérisé en ce qu'il comprend un polymère adhésif en émulsion suivant la revendication 1.